# EUROPEAN PATENT APPLICATION

(11) **EP 4 272 783 A2**
(43) Date of publication of application: **08.11.2023**
(21) Application number: 23199011.0
(22) Date of filing: 03.04.2020
(51) Int. Cl.: A61M 5/315

(54) **DOSE DELIVERY MECHANISM WITH SPINNING THROUGH PREVENTION**

(30) Priority: 11.04.2019 US 201962832636 P
(62) Divisional of application: 20717609.0
(71) Applicant: medmix Switzerland AG, 9469 Haag (Rheintal) (CH)
(72) Inventor: KEITEL, Joachim, 73728 Esslingen (DE)
(74) Representative: Manitz Finsterwald Patent- und Rechtsanwaltspartnerschaft mbB

(57) **Abstract**

An injection device incorporating a dose delivery mechanism is presented where a spinning through prevention feature is employed to maintain dose accuracy. The spinning through prevention feature can take of the form of a brake to prevent a rotating driver from inducing rotation of a nut during dose delivery. Alternatively, the spinning through prevention feature can employ freewheel design along with nut and ratchet assembly that can move from a first to a second position.

## Description

### TECHNICAL AREA

The present disclosure relates to a dose setting and dose delivery mechanism for an injection device, where the dose delivery mechanism is designed and configured to prevent a spinning through failure mode where a nut threaded to a piston rod rotates during dose delivery. Preventing the nut from rotating ensures dose accuracy.

### BACKGROUND

There are a number of medicament delivery devices on the market that are capable of automatically, semi-automatically or manually delivering doses of medicament. Of the known type of delivery devices the "pen-type" injector has gained in popularity and is available in both reusable and disposable designs. Such devices can be designed and constructed with dose setting and delivery mechanisms that employ a non-rotating piston rod, where a desired or predetermined dose is set by a user such that a nut in threaded engagement with the piston rod translates distally along the nut in an amount proportional to the set dose. Design features attempt to prevent the nut from rotating about the piston rod upon dose delivery, but in some instances high delivery forces can cause a spinning through failure mode where the nut rotates as the nut is push proximally.

After a user sets dose of medicament, a driver is typically activated such that the nut is pushed proximally, hopefully without rotation relative to the dose delivery mechanism housing. Since the nut is in threaded engagement with the piston rod, the axial movement of the nut should in theory only causes an equal amount of axial movement of the piston rod in the proximal direction. This axial movement of the piston rod causes the dispense of the set dose of medicament from a medicament container in the injection device. It is essential that the nut does not rotate relative to the piston rod during dose delivery because this would greatly affect dose accuracy. Stated another way, when the user sets a dose, the nut is displaced a linear distance along the outside of the piston rod. This distance defines and is directly proportional to the set dose of medicament that user intends be delivered. If the nut is allowed to rotate back down the outside of the piston rod during dose delivery, the original set linear distance will be decreasing as the nut and the piston rod move proximally. As such, when the nut finally encounters a hard stop, the distance traveled by the piston rod within the medicament container will be less than the linear distance that originally existed when the dose was set. This less traveled distance causes less medicament to be dispensed, i.e., less than the dose initially set, thus greatly reducing dose accuracy. Importantly, a user of the device may not notice this less traveled distance and as such would not be aware that less than the set dose of medicament was actually delivered to an injection site. Clearly this could lead to significant health issues because of this under dosing.

One cause of the undesirable nut rotation is when the driver is rotated relative to the nut during dose delivery, inducing and exerting a rotational force on the distal end of the nut as the driver pushes the nut forward proximally. In some designs, if this rotational force is great enough, it may cause slippage of the nut relative to a ratchet that nominally is designed to hold the nut in a non-rotational position during dose delivery. If the forces exerted by a user are high enough the driver can cause the rotation of the nut relative to the piston rod as the nut and piston rod move proximally. As such there is a need in the design of injection devices to configure a dose delivery mechanism to prevent rotation of the piston rod during dose delivery.

This disclosure is directed to dose delivery mechanism designs that significantly inhibit and, in some cases, totally prevent rotation of the nut relative to the piston rod during dose delivery. Although there exist many drug delivery devices available for patient use, there is clearly a need to ensure dose accuracy through application of mechanical designs that prevent driver induced rotation of a nut that is in threaded engagement with a non-rotating piston rod.

The present disclosure provides a solution to the above-mentioned concerns and problems with existing medicament delivery devices and provides an injector design that fulfills the needs and requirements mentioned above.

### SUMMARY

Some injection devices are manufactured and designed as so-called fixed dose designs where the dose dial sleeve contains printing signifying only one or two doses. The design idea behind these devices is for the user is to rotate the dose setting knob until one of the fixed dose setting is observed, typically in a window of the injector housing. Other injection device designs allow a user to dial (set) a dose that is selected from a plurality of possible doses as indicated on a rotating dose dial sleeve having a printed scale that is visible through a window in the housing of the dose setting mechanism. In most types of pen-type injector designs, a dose setting and delivery mechanism is located at the distal end of the injection device and a medicament container, such as, a cartridge, is located in the proximal end. The known injector designs typically are multiple (variable) dose devices, meaning that the user can select (dial) a dose between 0 and a maximum allowable settable dose. Typically, a dose dial sleeve is printed with a range of possible dose settings that correspond to each possible incremental dose setting.

To deliver a set dose the user will typically exert an axial force in a proximal direction relative to the housing by pressing on a dose knob, which was turned by the user to set a dose. Typically, rotation of the dose knob during dose setting will cause simultaneous rotation of a nut threadedly engaged with the piston rod. This simultaneous rotation can be achieved through a clutch mechanism.

If the thread between nut and piston rod is not self-locking, the exerted force applied by a user or by a pre-tension drive spring, could result in an applied pressure on the nut that could potentially lead to an unwanted and non-desirable rotation of the nut relative to the piston rod, causing a so-called spinning through failure mode during dose injection (delivery). This spinning through can cause the nut to start turning and spiralling down the piston rod, effectively reducing the previously set dose. Depending on the severity of the spinning through effect that occurs, the amount of expelled medicinal product can be greatly reduced and, in some cases, can even result in no dose being delivered. The force during dose delivery that is transmitted to the driver will be high, especially when the user injects fast. Likewise, if the medicament has a relative high viscosity or if the needle cannula or other conduit becomes obstructed or blocked, the user will press the dose knob with all the thumb force the user has to complete the injection, and the force transmitted to the driver can become very high. As such, the design of the dose delivery mechanism must be robust enough that the nut, which rotates during dose setting, does not slip and rotate during injection, even if the thread is not self-locking and even if the force is high.

In prior US 8,747,367, incorporated herein in its entirety, the nut has one or more flexible arms, which interfere with splines of the pen body. During dose setting, those flexible arms jump over those splines to define the intended doses while also providing an audible and tactile feedback (i.e., clicks) signifying to a user that a dose is being dialled. In some design configurations to accommodate users that want or need to exert a lower dose setting torques, then the spinning through problem becomes more evident. To address these situations, the click assembly design can be modified to provide a spinning through prevention feature as outlined in the present disclosure, where the torque on the nut induced by the force of the driver during injection is reduced and minimized such that the flexible arms do not jump over the splines resulting in relative rotation of the nut to the piston rod. In other words, preventing counter rotation of the nut relative to the piston rod during dose delivery and thus preserving dose accuracy. The design disclosed in prior US 9,694,136, also incorporated herein in its entirety, presents a partial solution to the problem through the use of a freewheel, where the nut can rotate in one direction (e.g., clockwise) to set a dose, but cannot rotate in the opposite direction (e.g., counter-clockwise). Employing such a partial solution, however, results in an undesirable side effect where the user cannot reduce the dose setting after unintentionally over dialling the dose knob, i.e., there is no way to correct a dose setting or completely cancelling a dose.

In designs where the driver only moves axially (e.g., see US 8,747,367) the spinning through problem is less likely to occur because the driver is non-rotating and only exerts an axial force that pushes the nut axially in the proximal direction. In other words, there is no rotational driving force to cause the nut to rotate relative to the piston rod. However, is some designs, the driver is rotated as it translates and pushes the nut proximally. Such rotation is caused by the engagement of the driver with the dose dial sleeve during dose delivery. Embodiments of this design can be found in prior US 9,694,136 B and WO 2019/011394, again incorporated herein in its entirety.

The present disclosure provides at least two dose delivery mechanism designs that greatly reduce or completely eliminate the spinning through failure mode that can occur when a spiralling driver design is employed. In a first possible embodiment of the present disclosure, a dose delivery mechanism is disclosed having housing that contains a non-rotating piston rod with a threaded outer surface. The piston rod could have a non-circular cross-section that is held non-rotational relative to the housing by a piston rod guide that is also rotationally fixed relative to the housing. A nut, having an inner surface that is in threaded, can be engaged with the threaded outer surface of the piston rod, where the nut can rotate and translate axially in a proximal direction relative to the piston rod during dose setting. Rotation of the nut during dose setting can be accomplish through a clutch that is operatively connected to a dose knob that is rotated by a user of the dose setting mechanism. The nut could have one or more flexible arms that releasably engage a ratchet during dose setting and dose cancellation. The relative movement and engagement of the flexible arm with the ratchet could generate tactile or audible signals that the user would sense.

A driver having an outer surface and configured to rotate during dose delivery can also be part of the presently disclosed dose setting mechanism. The ratchet can be rotationally fixed relative to the housing, but configured for axial movement during dose setting and dose delivery. The ratchet, as described above, can be operatively connected to a proximal end of the nut through one or more flexible arms having nibs that engage splines in the ratchet. A spinning through prevention feature is located between the nut and the ratchet. In some configurations, where the driver does not rotate during dose delivery, then the spinning through prevention feature could be positioned between the driver and the nut. The prevention feature would then prevent inadvertent rotation to the nut, thereby preventing the nut from rotating relative to the stationary piston rod.

The driver can have an outer surface that is configured to cause it to rotate during dose delivery. The outer surface could contain splines that could engage cooperating splines on the inside of a dose dial sleeve, such that the sleeve and the driver are rotationally fixed to each other. When the dose dial sleeve spirals back down into the housing during dose cancelation or dose delivery, the driver likewise rotates and translates proximally relative to the housing. Depending on the configuration, the axial movement of the driver as it translates proximally will exert forces directly on the ratchet or directly on the nut. The presence of the brake ensures that only a linear and axial pushing of the nut occurs in the proximal direction relative to the housing. When the driver exerts forces directly on the ratchet, the axial motion of the ratchet is transmitted to the nut and the threadedly connected piston rod such that the nut and piston rod also move only axially during dose delivery.

The brake is preferably positioned adjacent a distal surface of the nut and adjacent a terminal proximal end of the ratchet. However, in some dose setting mechanism designs, the ratchet is not a separate component and is instead part of the inner surface of the housing. In such designs the brake can be positioned between the driver and the nut. The brake can be a ring or washer like structure having a through hole to accept a proximal end of the nut and can be made of rubber that should be rigid enough such that is does not compress significantly as a result of the axial forces applied to the driver during dose delivery. Yet, the brake must be flexible enough to work as a brake to prevent imparting rotation onto the nut from the driver. The surfaces of the ratchet and the nut that are adjacent and facing the top and bottom of the brake could have a fine textural structure, which catches or engages in the brake material under pressure, but glide over the brake without pressure, such as in dose cancellation or dose setting. After an injection (dose delivery) is completed, the force between ratchet and nut will be significantly lower than during the injection procedure. As such, the user will be able to set a second or new dose without being hindered, restricted or prohibited by the brake.

In configuration where the driver directly engages the ratchet, during the dose delivery procedure the driver rotates relative to the ratchet while pushing the ratchet axially in the proximal direction. The interface between the ratchet and the driver is responsible for a considerable fraction of the friction during does delivery. To minimize or eliminate most of the friction forces, it can be beneficial to us a glider that is positioned between the ratchet and the rotating driver. Such a glider could, for example, be a washer made of Teflon or other like low friction material.

In another possible dose delivery mechanism design a combination of a freewheel feature and changing nut positions can be incorporated into a dose delivery mechanism to prevent the spinning through failure mode from occurring. Such an embodiment also has housing, a non-rotating piston rod having a threaded outer surface, a nut having an inner surface in threaded engagement with the threaded outer surface of the piston rod, where the nut rotates and translates axially in a proximal direction relative to the piston rod during dose setting. A piston rod guide can be rotationally fixed relative to the housing and configured to prevent rotation of the piston rod. A driver is positioned within the housing and having an an outer surface configured such that the driver rotates and moves proximally during dose delivery. A ratchet can be rotationally fixed to relative to the housing and be operatively engaged with a proximal end of the nut such that the nut can move from a first position where dose cancellation is prevented to a second position where dose cancellation is possible. The ratchet can also have an inner surface with a radial lip that interacts with the nut as the nut moves from the first position to the second position. The ratchet also may have a linear guide radially extending from an outside surface such that the linear guide is engaged with the piston rod guide to prevent rotation of the ratchet relative to the housing.

A glider can be positioned between the driver and the nut to minimize frictional forces caused by the relative rotation of the driver relative to the nut. The driver outer surface can be configured to be operatively connected to the inside surface of a dose dial sleeve such that upon dose delivery or dose cancellation the driver rotates and moves axially in the proximal direction. Translation of driver pushes the nut axially in a proximal direction relative to the housing. A proximal end of the nut can have a radial flexible arm that engages the ratchet when nut is in the first position, where the radial arm comprises a nib that when engaged with the ratchet only allows for rotation of the nut in a first direction and prevents rotation of the nut in second opposite direction. When the nut is in the second position, the radial flexible arm that does not engage the ratchet splines and therefore the nib no longer prevents rotation of the nut relative to the piston rod or the housing. The nut can also have a positioning protrusion that engages the radial lip on the inside of the ratchet when the nut is in the second position. This keeps the nut in the second position at an axial distance distal to the first position thus preventing the flexible arm on the nut from engaging the ratchet splines.

The dose setting mechanism of this disclosure also can contain a clutch that is operatively connected to the dose knob at a distal end of the clutch. In one embodiment, the proximal end of the clutch is rotationally fixed to a nut and is axially slidable relative to the nut. The nut can be threadedly engaged with a piston rod that is configured to move only axially in the proximal direction such that during dose delivery the piston rod exerts an axial force causing a plunger within the container of medicament to move proximally pressurizing the medicament so that it is discharged through a proximal opening in the medicament container. A preferred shape of the piston rod includes one having a non-circular cross-section and having threads on the outside surface. The pitch of these threads is directly proportional to each dialed dose setting or predetermined fixed dose of medicament. A piston guide having a non-circular center opening can be included in the dose setting mechanism, where the piston guide accepts the non-circular cross-section of the piston rod such that the piston guide prevents the piston rod from rotating during both dose setting and dose delivery.

The dose setting knob and clutch can be operatively connected such that they are rotationally fixed to each other so that during dose setting rotation of the dose knob rotates the clutch, which in turn rotates the nut. Rotation of the nut causes the nut to translate axially in a distal direction along threads located on the outer surface of the piston rod during dose setting and to translate in the proximal direction during dose cancellation. During dose delivery, it is desired that the nut move only axially with the piston rod a distance in a proximal direction. This distance is directly proportional to the set dose. This axial only movement of the nut necessarily causes axial movement of the piston rod because of the threaded engagement with the nut. As mentioned, during dose setting the axial translational movement of the nut in the distal direction is directly proportional to an amount of the medicament that would be delivered if the piston rod was then moved proximally without rotation of the nut relative to the piston rod.

This disclosure is also directed to complete injection devices. One possible embodiment of such an injection device includes a dose setting mechanism as described having a connector mechanism at a proximal end configured to connect with and attach to a holder configured to accept a container, preferably a cartridge, containing a medicament to be delivered to a patient in a series of set doses or in a single dose. A dose setting mechanism as described above can be used in this injection device where the dose selector is configured to allow only a set of finite predetermined fixed doses to be set by a user of the device, where the set of finite predetermined fixed doses includes a lowest fixed dose and one or more higher fixed doses. In some configurations, at least one of the one or more higher fixed doses could be equal to the lowest fixed dose plus a fractional amount of the lowest fixed dose.

These and other aspects of, and advantages with, the present disclosures will become apparent from the following detailed description of the present disclosure and from the accompanying drawings.

### BRIEF DESCRIPTION OF THE FIGURES

In the following detailed description of the present disclosure, reference will be made to the accompanying drawings, of which
- Fig. 1A: illustrates one possible embodiment of a complete injection device containing the structural components of the present disclosure;
- Fig. 1B: shows a cross-sectional view of just the distal end or dose delivery mechanism of the injection device of Fig. 1A;
- Fig. 1C: shows a cross section view of a portion of the dose delivery mechanism of Fig. 1B;
- Fig. 2A: shows perspective views of some of the structural components of one of the possible dose delivery mechanisms of the present disclosure;
- Fig. 2B: shows a cross section of the dose delivery components of Fig. 2A;
- Fig. 2C: shows a cross section of a portion of the components of Fig. 2B at B-B;
- Fig. 3: shows perspective views of some of the structural components of another embodiment of the dose delivery mechanisms of the present disclosure;
- Fig. 4: shows a cross section of the dose delivery components of Fig. 3 in a first position;
- Fig. 5: shows a cross section of a portion of the components of Fig. 4 at B-B;
- Fig. 6: shows a cross section of the dose delivery components of Fig. 3 in a second position;
- Fig. 7: is a perspective view of a possible nut of the present disclosure the piston rod;
- Fig. 8A: is a perspective view of the of one embodiment of a ratchet;
- Fig. 8B: is a cross section of the ratchet shown in Fig. 8A; and
- Fig. 8C: is a top view of the ratchet shown in 8A.

### DETAILED DESCRIPTION

In the present application, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the members thereof, which in accordance with the use of the device is located closest to the delivery/injection site of the patient.

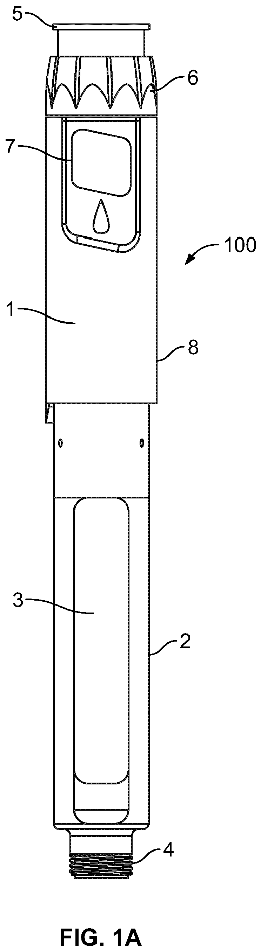

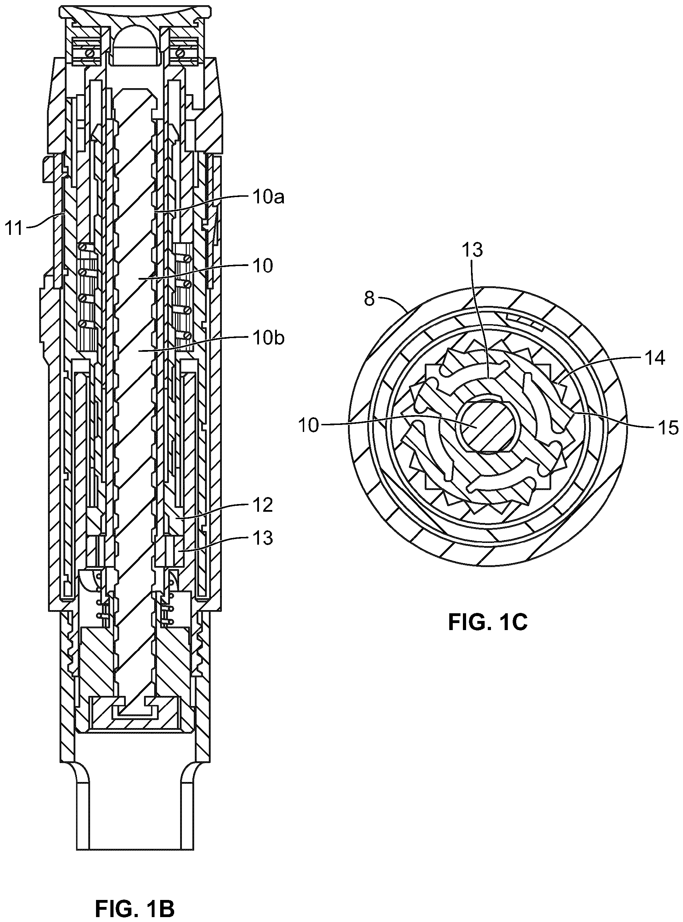

The dose delivery mechanism of the present disclosure can be used in a number of variously designed complete injection devices. One such embodiment of a complete injection device 100 is illustrated in in Fig. 1, where a dose delivery mechanism 1 is connected to a medicament container holder 2, which holds a medicament container 3, preferably a cartridge. The container holder has a proximal end with connector 4 configured to accept a medicament delivery conduit, preferably a double ended needle cannula (i.e., a pen needle). The pen needle is attached to connector 4 through a snap fit, thread, Luer-Lok, or other secure attachment with a hub such that the double ended needle cannula can achieve a fluid communication with medicament contained in cartridge 3. The cartridge 3 is sealed at the proximal end by septum and with a sliding piston at the opposite distal end. Dose setting are shown in by indicia in window 7 of housing 8 of the dese delivery mechanism 1. Fig. 1 shows the delivery device where a protective cap covering the medicament container holder 2 is removed. A dose is set (or cancelled) when a user turns dose knob 6. A dose is delivered when a user presses dose button 5 after a dose has been set causing the dose knob 6, dose dial sleeve 11 (see Fig. 1B), nut 13, piston rod 10 and driver 12 all to move proximally.

Fig. 1B shows a cross section of one possible embodiment of the dose setting and dose delivery mechanism 2 of the present disclosure. Fig. 1C is a cross sectional view of the dose delivery mechanism shown in Fig. 1B, where a flexible arm 15 at the proximal end of nut 13 is shown engaged with ratchet 14. Piston rod 10 can also have a threaded outer surface 10a and have a non-circular cross section.

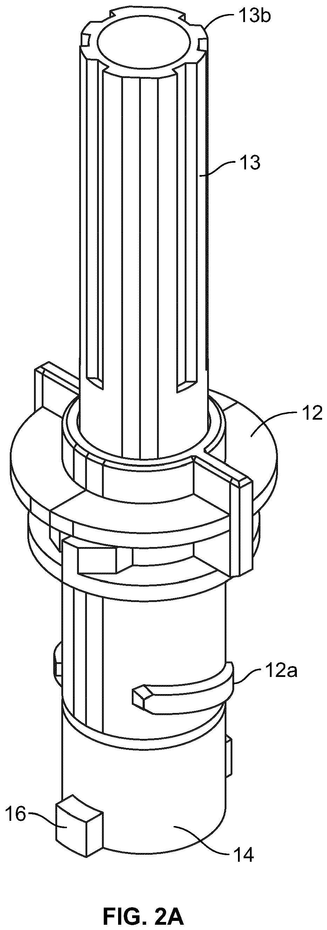

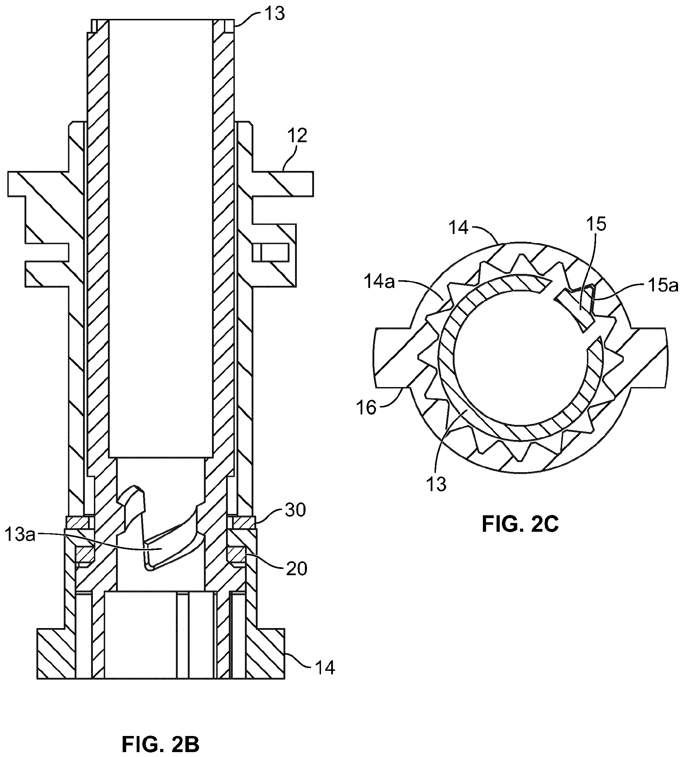

In another possible embodiment, as illustrated in Fig. 2A-2C, there is shown a nut 13 having an outer surface containing longitudinal splines 13b that engage and rotationally lock the nut to a clutch such that rotation of the dose knob causes rotation of the nut 13. Nut 13 also has a threaded inner surface 13a that is threadedly engaged with threads 10a on the outer surface of the piston rod 10. Driver 12 has an outer thread 12a that cooperates with a threaded inner surface of a piston guide that is rotationally fixed relative to the housing 8. A brake 20 is positioned between the nut 13 and the ratchet 14 to provide a spinning through prevention feature. The driver 12 is rotationally fixed to the dose dial sleeve 11, which rotates during dose setting, dose cancelation and dose delivery. The piston rod guide can have a non-circular through hole that slidably accepts the piston rod and prevents the piston rod from rotating during dose setting and dose delivery. A glider 30 may be positioned between the driver and the ratchet to reduce or eliminate the friction caused by the rotating driver during dose delivery. Ratchet 14 is held non-rotational relative to the housing 8 by the linear guide 16, which can be configured to engage the piston rod guide.

Fig. 2C shows one example of a flexible arm 15 located on the proximal end of nut 13 engaged with an inner surface of ratchet 14. This design of flexible arm 15 has a nib 15a that is configured to releasably engage splines 14a inside of ratchet 14 as the nut 13 is rotated in either direction. Although only one flexible arm is shown, multiple flexible arms can be used. The releasable configuration of the nib with the ratchet splines allows the user to conveniently correct a miss-dialed dose by rotating the dose knob in the opposite direction. The nut 13 and the clutch are permanently splined to each other during assembly of the dose delivery mechanism through a splined connection. This splined connection ensures that the clutch and nut are always rotationally fixed to each other during both dose setting and dose delivery. This splined connection also allows the clutch and the nut to move axially relative to each other. The sliding connection is necessary in order to compensate for pitch differences between the threads 10a on the piston rod 10, the outer threads on the dose sleeve and the thread 12a on the driver 12. Preferably, the thread between driver and piston rod guide has basically the same pitch as the thread between piston rod and nut.

As shown in Fig. 1B, in addition to threads 10a on the outer surface of the piston rod 10, there is also included two longitudinal flats 10b that give piston rod 10 a non-circular cross section. At the terminal proximal end of the piston rod is a connector, shown as a snap fit, that connects with a disc or foot. At the distal end of piston rod there can be a last dose feature of the dose setting mechanism configured as an enlarged section of the piston rod designed to stop the rotation of nut 13 about threads 10a when the amount of medicament remaining in the cartridge 3 is less than the next highest predetermined dose setting. In other words, if the user tries to set one of the predetermined fixed dose settings that exceeds the amount of medicament remaining in the cartridge, then the enlarged section will act as a hard stop preventing the nut from further rotation along threads 10a as the user attempts to reach the desired predetermined fixed dose setting.

In a possible embodiment, a rotational biasing member, for example torsion spring, could be connected to the driver 12, which is connected and rotationally fixed with the inner surface of dose sleeve through splines on the distal outer surface of the driver. On the proximal end of driver 12 on the outer surface is threads 12a that are engaged with matching threads on the inner distal surface of the piston rod guide. The thread between driver and piston guide has a significantly different pitch than the thread between dose sleeve and housing. The nut and the driver rotate together both during dose setting and dose cancellation and, as such, they perform essentially the same axial movement. However, this movement is independent from each other, i.e., the nut is turned by the clutch and performs an axial movement due to the thread to the piston rod, while the driver is rotated by the dose sleeve and performs an axial movement due to the thread to the piston guide. The driver is rotating during injection also, and so it actively moves in the proximal direction during injection. The nut does not rotate during injection and, as such, does not perform an active axial movement. The nut is only moving axially in the proximal direction during injection because it is being pushed axially by the driver as it rotates. To prevent the rotating driver from inducing a rotational motion of the piston rod (i.e., spinning through) the brake 20 is employed to provide a friction surface to ensure that the piston rod only moves in an axial direction, thus preserving dose accuracy.

It is preferred that the pitch of the thread on the driver is equal to or be slightly higher than the pitch of the thread on the inside of the nut. And, the thread between the dose sleeve and the housing has a higher pitch than that of the nut and piston rod. This is desirable because it yields a mechanical advantage that makes the dose delivery process easier for the user.

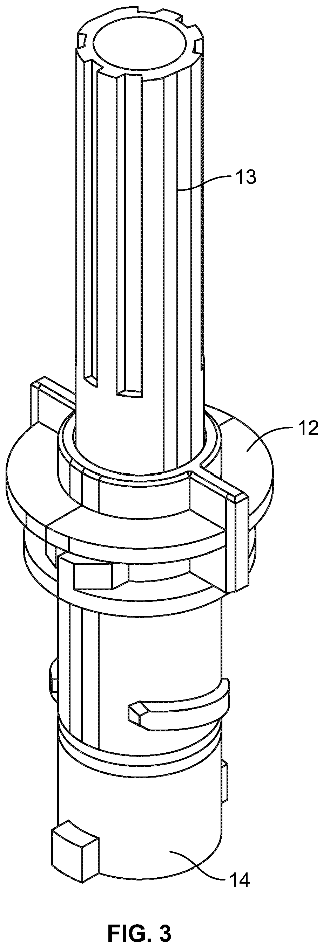

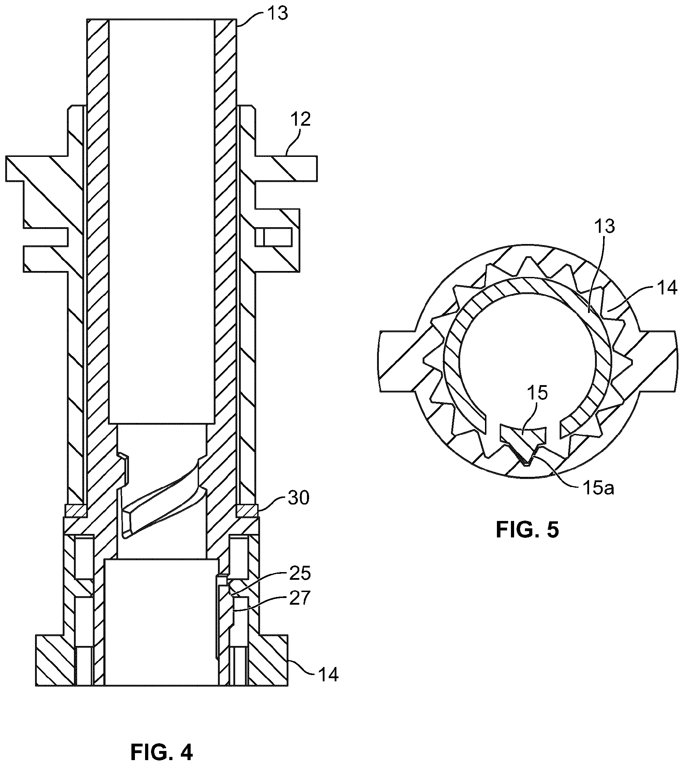

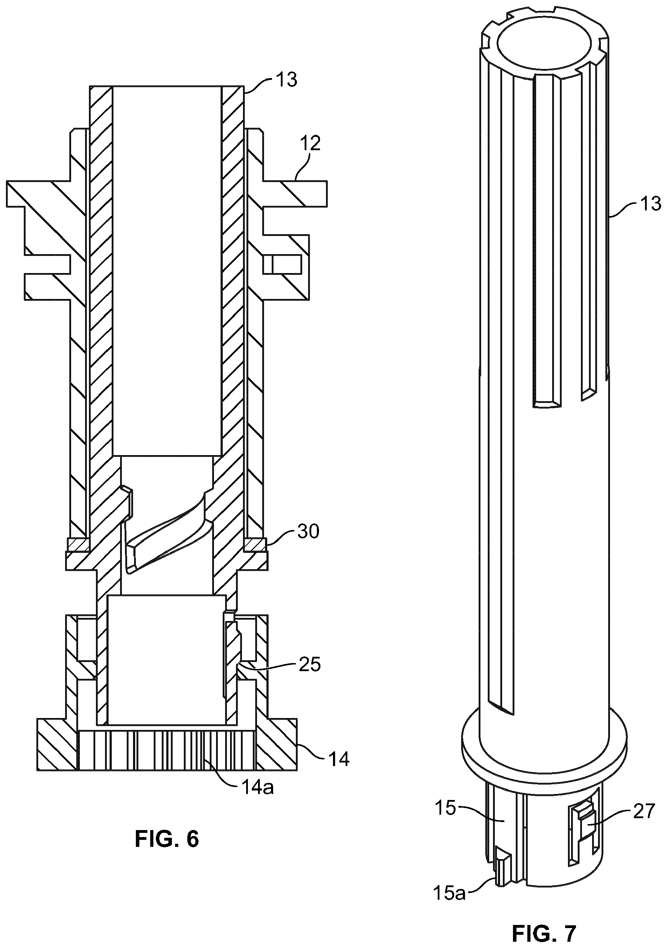

Another embodiment of a dose delivery mechanism to prevent a spinning through failure mode is illustrated in Figs. 3-8C, where a free wheel design is employed in combination with a two position nut and ratchet assembly . In this embodiment, the flexible arm 15 and nib 15a are configured such that the nut can only rotate in one direction when the nib is engaged with the splines on ratchet 14. This single direction of rotation of the nut 13 occurs during dose setting. The nut cannot be rotated during dose delivery so there is no issue regarding the failure mode of spinning through. However, such a design has the negative feature that dose cancelation is not possible. To allow dose cancellation this embodiment utilizes a two-position relationship between the nut and the ratchet. Figs. 4 & 5 shows the nut 13 in the first position. Fig. 6 shows the nut in a second position where the flexible arm 15 and nib 15a are no longer engaged with the splines 14a inside ratchet 14. During normal dose setting operation the ratchet and nut are in the relative axial position shown in Fig. 4. In this position the flexible arm of the nut interacts with the splines of the ratchet. The linear guide between ratchet and piston rod guide limits the linear movement of the ratchet. The distal end position of the ratchet is reached earlier than distal end position of the nut and the driver. If the user turns the dose knob beyond that distal end position of the ratchet, the ratchet and nut are pulled apart, so that they switch from the first position (Fig. 4) to the second position (Fig. 6). In the second position the flexible arm of the nut is not in contact to the splines of the ratchet and the user can turn the dose knob back to zero if the user has inadvertently dialled a dose larger than intended or wants to merely cancel a set dose. In the zero position the nut is pushed back into the ratchet, so that the components switch from second position back to the first position causing the flexible arm of the nut to again engage and interfer with the splines of the ratchet.

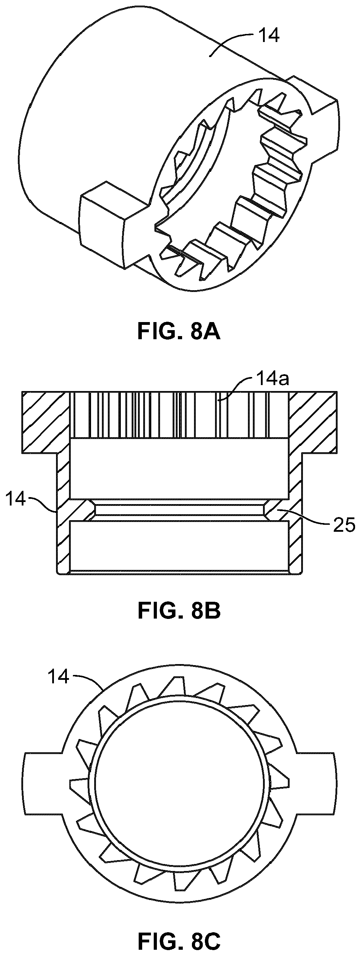

A positioning protrusion 27 located on the nut and a radial lip 25 located on the ratchet are operatively engaged to keep the nut in either the first or the second positions. This is evident by a comparison of Figs. 4 and 6. Fig. 7 shows the positioning protrusion 27 on nut 13 relative to the flexible arm 15. Figs. 8A-8C show the radial lip 25 on the inside surface of ratchet 14 and positioned relative to splines 14a. A glider 30 can be positioned between the driver 12 and nut 13.

It is to be understood that the embodiments described above and shown in the drawings are to be regarded only as non-limiting examples of the possible designs of the safety assembly and such designs may be modified in many ways within the scope of the patent claims.

The present disclosure relates to the following embodiments:
1. A non-slip dose delivery mechanism comprising:
   a housing;
   a non-rotating piston rod having a threaded outer surface;
   a nut comprising an inner surface that is in threaded engagement with the threaded outer surface of the piston rod, where the nut rotates and
   translates axially in a proximal direction relative to the piston rod during dose setting;
   a piston rod guide rotationally fixed relative to the housing and configured to prevent rotation of the piston rod;
   a driver comprising an inner surface and an outer surface, where the inner surface is in a rotationally fixed engagement with the nut;
   a ratchet rotationally fixed to relative to the housing; and
   a brake positioned between the nut and the ratchet.
2. The non-slip dose delivery mechanism of embodiment 1, wherein the piston rod has a non-circular cross-section that is held non-rotational relative to the housing by a piston rod guide that is rotationally fixed relative to the housing.
3. The non-slip dose delivery mechanism of embodiment 1, wherein rotation of the nut during dose setting is through a clutch that is operatively connected to a dose knob that is rotated by a user of the dose setting mechanism.
4. The non-slip dose delivery mechanism of embodiment 3, wherein the nut has one or more flexible arms that releasably engage the ratchet during dose setting and dose cancellation.
5. The non-slip dose delivery mechanism of embodiment 4, where relative movement and engagement of the one or more flexible arms with the ratchet generates a tactile or audible signal to a user of the non-slip dose delivery mechanism.
6. The non-slip dose delivery mechanism of embodiment 1, where the outer surface of the driver is configured to cause the driver to rotate and move axially during dose delivery.
7. The non-slip dose delivery mechanism of embodiment 1, where axial movement of the driver pushes the nut axially in a proximal direction relative to the housing.
8. The non-slip dose delivery mechanism of embodiment 1, where the brake is adjacent a distal terminal end of the driver and adjacent a terminal proximal end of the ratchet.
9. The non-slip dose delivery mechanism of embodiment 1, where the brake is a ring having a through hole to accept a proximal end of the nut.
10. The non-slip dose delivery mechanism of embodiment 1, where the ratchet is fixed to the piston rod guide and is fixed to an inside surface of the housing.
11. An injection device comprising the non-slip dose delivery mechanism of embodiment 1 and a holder attached to the non-slip dose delivery mechanism, where the holder is configured to accept a container of medicament.
12. An anti-slip dose delivery mechanism comprising:
   a housing;
   a non-rotating piston rod having a threaded outer surface;
   a nut comprising an inner surface that is in threaded engagement with the threaded outer surface of the piston rod, where the nut rotates and
   translates axially in a proximal direction relative to the piston rod during dose setting;
   a piston rod guide rotationally fixed relative to the housing and configured to prevent rotation of the piston rod;
   a driver comprising an inner surface and an outer surface, where the inner surface is in a rotationally fixed engagement with the nut;
   a ratchet rotationally fixed to relative to the housing and comprising an inner surface with a radial lip; and
   a glider positioned between the driver and the nut;
   wherein the nut can move from a first position where dose cancellation is prevented to a second position where dose cancellation is possible.
13. The anti-slip dose delivery mechanism of embodiment 12, where the driver has an outer surface configured to cause the driver to rotate and move axially during dose delivery.
14. The anti-slip dose delivery mechanism of embodiment 12, where the nut further comprises a radial flexible arm that engages the ratchet when nut is in the first position.
15. An injection device comprising the anti-slip dose delivery mechanism of embodiment 12 and a holder attached to the anti-slip dose delivery mechanism, where the holder is configured to accept a container of medicament.

## Claims

1. A non-slip dose delivery mechanism comprising:
a housing (8);
a non-rotating piston rod (10) having a threaded outer surface (10a);
a nut (13) comprising an inner surface (13a) that is in threaded engagement with the threaded outer surface (1 0a) of the piston rod (10),
wherein the nut (13) rotates and translates axially in a distal direction relative to the piston rod (10) during dose setting;
a driver (12) that does not rotate during dose delivery;
a ratchet (14) rotationally fixed relative to the housing (8); and
a brake (20) positioned between the nut (13) and the driver (12).

2. The non-slip dose delivery mechanism of claim 1,
wherein the brake (20) is a ring or washer like structure having a through hole to accept a proximal end of the nut (13).

3. The non-slip dose delivery mechanism of claim 2,
wherein the brake (20) is made of rubber.

4. The non-slip dose delivery mechanism of one of the preceding claims,
wherein, during dose delivery, the nut (13) moves only axially with the piston rod (10).

5. The non-slip dose delivery mechanism of one of the preceding claims,
wherein the piston rod (10) has a non-circular cross-section that is held non-rotational relative to the housing (8) by a piston rod guide that is rotationally fixed relative to the housing (8).

6. The non-slip dose delivery mechanism of one of the preceding claims,
wherein rotation of the nut (13) during dose setting is through a clutch that is operatively connected to a dose knob (6) that is rotated by a user of the dose setting mechanism.

7. The non-slip dose delivery mechanism of claim 6, wherein the nut (13) has one or more flexible arms (15) that releasably engage the ratchet (14) during dose setting and dose cancellation.

8. The non-slip dose delivery mechanism of claim 7, wherein relative movement and engagement of the one or more flexible arms (15) with the ratchet (14) generates a tactile or audible signal to a user of the non-slip dose delivery mechanism.

9. The non-slip dose delivery mechanism of one of the preceding claims,
wherein axial movement of the driver (12) pushes the nut (13) axially in a proximal direction relative to the housing (8).

10. The non-slip dose delivery mechanism of one of the preceding claims,
wherein the ratchet (14) is fixed to an inside surface of the housing (8).

11. The non-slip dose delivery mechanism of one of the preceding claims,
wherein the ratchet (14) is part of an inner surface of the housing (8).

12. An anti-slip dose delivery mechanism comprising:
a housing (8);
a non-rotating piston rod (10) having a threaded outer surface (10a);
a nut (13) comprising an inner surface (13a) that is in threaded engagement with the threaded outer surface (10a) of the piston rod (10), wherein the nut (13) rotates and translates axially in a distal direction relative to the piston rod (10) during dose setting; and
a ratchet (14) rotationally fixed relative to the housing (8),
wherein the nut (13) can move from a first position where dose cancellation is prevented to a second position where dose cancellation is possible.

13. The anti-slip dose delivery mechanism of claim 13, wherein the nut (13) further comprises a radial flexible arm that engages the ratchet (14) when the nut (13) is in the first position.

14. The anti-slip dose delivery mechanism of one of claims 12 and 13,
wherein the ratchet (14) is configured to move axially relative to the housing (8) during dose setting,
wherein the nut (13) is caused to move from the first position to the second position at a distal end position of the ratchet (14).

15. An injection device (100) comprising the non-slip dose delivery mechanism of one of claims 1 to 11 or the anti-slip dose delivery mechanism of one of claims 12 to 14 and a holder (2) attached to the non-slip dose delivery mechanism or the anti-slip dose delivery mechanism, respectively, wherein the holder (2) is configured to accept a container of medicament (3),
wherein, for example, the holder (2) holds the container of medicament (3).
